# EUROPÄISCHE PATENTSCHRIFT

(11) **EP 2 533 832 B1**
(45) Veröffentlichungstag und Bekanntmachung des Hinweises auf die Patenterteilung: **19.08.2015**
(21) Anmeldenummer: 11700082.8
(22) Anmeldetag: 10.01.2011
(51) Int. Cl.: A61M 5/162, A61M 39/22, A61J 1/14

(54) **Konnektor für medizinischen wirkstoff enthaltende behälter**
Connector for containers containing medical agents
Connecteur pour récipient contenant un agent actif médical

(30) Priorität: 08.02.2010 US 302192 P; 08.02.2010 EP 10152921
(43) Veröffentlichungstag der Anmeldung: 19.12.2012
(73) Patentinhaber: Fresenius Kabi Deutschland GmbH, 61352 Bad Homburg v.d.H. (DE)
(72) Erfinder: RAHIMY, Ismael, 61169 Friedberg (DE); BRANDENBURGER, Torsten, 61203 Reichelsheim (DE)
(74) Vertreter: Brandt, Maximilian
(86) Internationale Anmeldenummer: PCT/EP2011/050229
(87) Internationale Veröffentlichungsnummer: WO 2011/095373

(56) Entgegenhaltungen:
- WO-A1-2004/017852
- WO-A1-2006/062912
- FR-A1- 2 718 970
- US-A1- 2010 108 681

## Beschreibung

### Technisches Gebiet

Die Erfindung betrifft einen Konnektor für medizinischen Wirkstoff enthaltende Behälter, der es ermöglicht, Wirkstoff aus einem Behälter in einen anderen Behälter zu überführen.

### Stand der Technik

Die EP 2 095 805 A2 offenbart einen Konnektor für medizinischen Wirkstoff enthaltende Behälter, der einen ersten Anschlussbereich für den Anschluss eines Glasfläschchens, einen zweiten Anschlussbereich für den Anschluss eines flexiblen Beutels und eine axial bewegliches, in Form einer doppelseitig angeschärften Kanüle ausgebildetes Durchstechelement umfasst. Das Durchstechelement ist axial verschiebbar in einem ersten Gehäuseabschnitt eines Gehäuses des Konnektors gelagert. Der erste Gehäuseabschnitt ist teleskopartig verschiebbar auf einem zweiten Gehäuseabschnitt gelagert. In der Ausgangsstellung des Konnektor sind der erste Gehäuseabschnitt und der zweite Gehäuseabschnitt auseinandergezogen. Durch Anschluss einer Glasflasche im ersten Anschlussbereich und durch Ausüben eines Druckes auf die Glasflasche verschiebt sich der erste Gehäuseabschnitt gegenüber dem zweiten Gehäuseabschnitt. Eine im Hals der Glasflasche angeordnete, die Glasflasche verschließende Membran trifft auf das eine Ende des Durchstechelementes. Das Durchstechelement wird beim Vorschieben der Glasflasche mitgeführt, bis es mit seinem anderen Ende auf eine den flexiblem Beutel verschließende Membran trifft. Durch weiteres Vorschieben der Glasflasche wird sowohl die Membran auf der Seite des flexiblen Beutels als auch die Membran der Glasflasche von dem Durchstechelement durchstoßen. Ein Anschlag für die Bewegung des Durchstechelementes begrenzt die Bewegung des Durchstechelementes und gewährleistet, dass nicht nur die beutelseitige Membran, sondern auch die Membran der Glasflasche geöffnet wird.

Nachteilig an diesem Konnektor ist, dass dieser mit den beiden Gehäuseabschnitten bewegliche Teile umfasst, die gegeneinander abgedichtet werden müssen. Des Weiteren umfasst der Konnektor eine Vielzahl von Elementen, die die Herstellung des Konnektors aufwendig und teuer machen. Ein selbstabdichtender Male-Luer-Konnektor, der auch eine Vielzahl an Abdichtungen aufweist, ist in der WO 2006/062912 A1 beschrieben.

### Kurzbeschreibung der Erfindung

Der erfindungsgemäße Konnektor für medizinischen Wirkstoff enthaltende Behälter umfasst einen ersten Anschlussbereich für den Anschluss eines ersten Behälters, ein Führungselement, ein Durchstechelement und eine Trennwand, wobei das Führungselement einen für die Überführung eines medizinischen Wirkstoffes ausgebildeten Kanal aufweist, das Durchstechelement zumindest abschnittsweise in dem Kanal angeordnet ist und innerhalb des Kanals vom Führungselement axial beweglich geführt wird, und durch das Anschließen eines Behälters das Durchstechelement von einer Ausgangsstellung, in der das Durchstechelement die Trennwand nicht öffnet, in eine Endstellung, in der das Durchstechelement die Trennwand zum Überführen eines medizinischen Wirkstoffs öffnet, bewegbar ist.

Dadurch, dass das Führungselement sowohl den Kanal ausbildet, durch den ein Wirkstoff überführt werden kann, als auch ein Lager ausbildet, in dem das Durchstechelement axial beweglich verschiebbar ist, kann das Führungselement eine Doppelfunktion übernehmen. Hierdurch kann die Anzahl der Elemente des Konnektors reduziert und der Konnektor kostengünstig hergestellt werden. Auch kann auf diese Weise die Anzahl der Schnittstellen reduziert und das Handling des Konnektors verbessert werden. Der erfindungsgemäße Konnektor ermöglicht insbesondere eine Überführung des medizinischen Wirkstoffes ohne Kontaminationsrisiko.

Die Behälter, die durch den Konnektor verbunden oder angeschlossen werden können, können sowohl geschlossene Behälter, beispielsweise Glasflaschen, Kunststoffflaschen oder Beutel, insbesondere flexible Beutel, oder "offene" Behälter, beispielsweise Katheter oder sonstige Leitungen, sein, wie sie beispielsweise in der Infusion, Transfusion, der klinischen Ernährung, Onkologie, Dialyse oder anderen medizinischen Feldern verwendet werden. Der medizinische Wirkstoff, der mittels des Konnektors von einem Behälter zum anderen Behälter übertragbar ist, kann beispielsweise eine Flüssigkeit oder aber auch ein Pulver sein.

Die Trennwand kann eine flexible Trennwand sein, beispielsweise aus einem elastischen Material, insbesondere in Form einer Membran, aber auch, was bevorzugt ist, eine steife Trennwand, die durch das Durchstechelement aufgebrochen oder durchstochen wird.

In eine bevorzugten Ausführungsform sind das Durchstechelement und das Führungselement derart ausgebildet sind, dass zwischen einer Außenfläche des Durchstechelementes und einer den Kanal bildenden Innenfläche des Führungselementes ein Freiraum für die Überführung eines medizinischen Wirkstoffes verbleibt. In einer alternativen Ausführungsform sind das Durchstechelement und das Führungselement derart ausgebildet, dass die Überführung des medizinischen Wirkstoffes zusätzlich oder ausschließlich durch das Durchstechelement erfolgt. Das Durchstechelement kann zu dem jeweiligen Zwecke massiv oder als Hohlkörper ausgebildet sein. Eine Kombination von Hohlkörper und massiven Körper ist grundsätzlich ebenfalls möglich. Das Profil des Durchstechelementes ändert sich vorzugsweise entlang seiner Achse, wobei der der Trennwand zugewandte Endabschnitt zum Aufbrechen oder Durchstechen der Trennwand ausgebildet ist, und der dem anzuschließenden Behälter zugewandte Endabschnitt derart ausgebildet ist, dass mittels des Behälters auf den Endabschnitt ein Druck ausübbar ist, durch den das Durchstechelement gegen die Trennwand gedrückt werden kann. Zu diesem Zweck kann der der Trennwand zugewandte Endabschnitt beispielsweise spitz zulaufend mit einem linienförmigen oder kreuzförmigen Profil ausgebildet sein, der andere Endabschnitt unter Ausbildung einer Auflagefläche mit beispielsweise einem kreuzförmigen oder runden Profil ausgebildet sein. Im Falle, dass das Durchstechelement als Hohlkörper ausgebildet ist, kann der der Trennwand zugewandte Endabschnitt mit einem Facettenschliff ausgebildet sein oder eine eingehämmerte Spitze aufweisen,

In einer bevorzugten Ausführungsform sind das Führungselement und die Trennwand einteilig, in einer besonders bevorzugten Ausführungsform einstückig ausgebildet.

Erfindungsgemäß umfasst der Konnektor ein den ersten Anschlussbereich ausbildendes Gehäuse. Vorzugsweise sind Gehäuse, Führungselement und Trennwand einteilig, besonders bevorzugt einstückig ausgebildet. Durch das einteilige und gegebenenfalls einstückige Ausbilden mehrerer funktioneller Elemente lässt sich die Anzahl der Teile des Konnektors reduzieren und lassen sich die Herstellungskosten reduzieren.

In einer weiteren bevorzugten Ausführungsform sind Durchstechelement, Führungselement, Trennwand und/oder Gehäuse aus einem Kunststoff, vorzugsweise Polypropylen (PP) oder einem Blend aus Polypropylen und Styrol/Ethylen-Butylen/Styrol (SEBS), hergestellt. Insbesondere können Durchstechelement, Führungselement, Trennwand und/oder Gehäuse Spritzgussteile sein. Das Durchstechelement kann alternativ aus beispielsweise Polycarbonat (PC) oder Polystyrol (PS) hergestellt sein.

Erfindungsgemäß umfasst das Führungselement einen Endabschnitt, der zum Durchstechen einer Membran eines in den ersten Anschlussbereich anschließbaren Behälters ausgebildet ist, beispielsweise zum Durchstechen eines Gummistopfens eines Glasfläschchens. Das Führungselement umfasst damit eine weitere Funktion. Auf ein weiteres Element, das zum Durchstechen einer Membran vorgesehen ist, kann auf diese Weise verzichtet werden.

In einer weiteren bevorzugten Ausführungsform ist der erste Anschlussbereich als Luer-Anschluss eines Luer-Locks ausgebildet, insbesondere als Male-Teil. Das Führungselement wird in diesem Falle teilweise oder vollständig durch den zentralen Konus des Luer-Anschlusses gebildet. Durch Anschließen des Female-Teils wird vorzugsweise beim Verschrauben der Verbindung das Durchstechelement aus seiner Ausgangsstellung in die Endstellung gedrückt, in der die Trennwand geöffnet ist. Der Anschluss des Gegenparts des Luer-Anschlusses ist damit mit einem Öffnen der Trennwand verbindbar, ein zusätzlicher Bewegungsablauf kann dadurch entfallen. Zudem wird das Risiko einer Kontamination gesenkt.

Weitere vorteilhafte Ausführungsformen der Erfindung sind Gegenstände der abhängigen Ansprüche.

Die Erfindung wird im Folgenden anhand von Ausführungsbeispielen, die durch mehrere Figuren dargestellt sind, näher erläutert.

### Kurze Beschreibung der Zeichnungsfiguren

Es zeigt:
Fig. 1 einen Längsschnitt durch eine erste Ausführungsform eines erfindungsmäßen Konnektors,
Fig. 2 eine Schnitt durch den in Fig. 1 gezeigten Konnektor entlang der Schnittlinie C-C.
Fig. 3 eine erste perspektivische Ansicht des in der Fig. 1 gezeigten Konnektors,
Fig. 4 eine zweite perspektivische Ansicht des in der Fig. 1 gezeigten Konnektors,
Fig. 5 einen Abschnitt des Durchstechelements des in der Fig. 1 gezeigten Konnektors,
Fig. 6 eine perspektivische Ansicht des entlang der Längsachse aufgeschnittenen, in Fig. 1 gezeigten Konnektors in einem Ausgangszustand,
Fig. 7 eine perspektivische Ansicht des entlang der Längsachse aufgeschnittenen, in Fig. 1 gezeigten Konnektors vor Anschluss eines Glasfläschchens,
Fig. 8 eine perspektivische Ansicht des entlang der Längsachse aufgeschnittenen, in Fig. 1 gezeigten Konnektors mit dem Glasfläschchen in einer Andockposition,
Fig. 9 eine perspektivische Ansicht des entlang der Längsachse aufgeschnittenen, in Fig. 1 gezeigten Konnektors mit dem Glasfläschchen in angeschlossener Endposition,
Fig. 10 eine perspektivische Ansicht des ersten Anschlussbereichs des in Fig. 1 gezeigten Konnektors,
Fig. 11 eine Seitenansicht einer zweiten Ausführungsform eines erfindungsgemäßen Konnektors mit einem an den Konnektor angeschlossenen Luer-Female-Teil,
Fig. 12 eine perspektische Ansicht des in Fig. 11 gezeigten Konnektors mit Luer-Female-Teil,
Fig. 13 eine Aufsicht des in Fig. 11 gezeigten Konnektors,
Fig. 14 einen Längsschnitt durch den in Fig. 11 gezeigten Konnektors mit Luer-Female-Teil,
Fig. 15 eine vergrößerte Darstellung des in Fig. 14 gekennzeichneten Ausschnitts A ,
Fig. 16 eine perspektivische Ansicht des entlang der Längsachse aufgeschnittenen, in Fig. 11 gezeigten Konnektors vor Anschluss des Luer-Female-Teils,
Fig. 17 eine perspektivische Ansicht des entlang der Längsachse aufgeschnittenen, in Fig. 11 gezeigten Konnektors mit angeschlossenem Luer-Female-Teil,
Fig. 18 eine perspektivische Ansicht des Durchstechelementes des in Fig. 11 gezeigten Konnektors.

### Beschreibung der Ausführungsarten

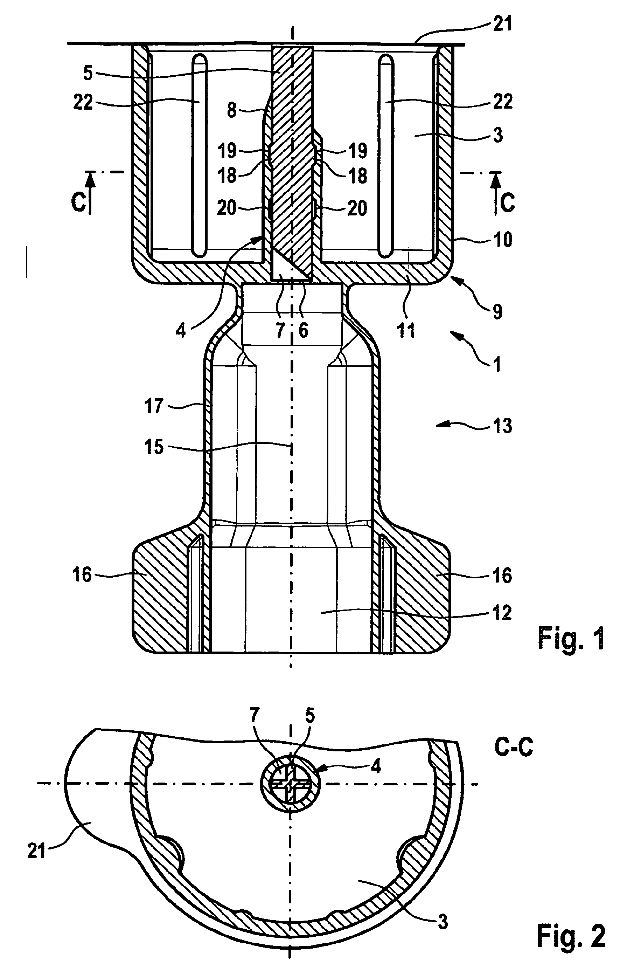

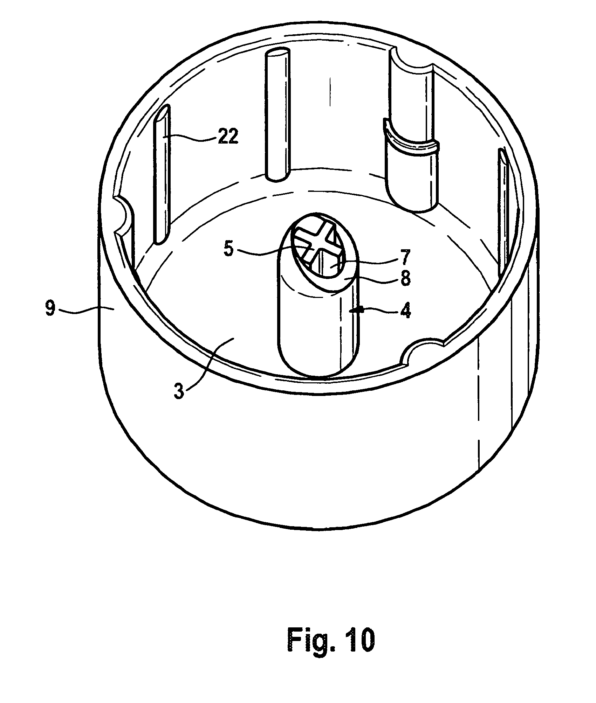

Die Fig. 1 bis 10 zeigen in verschiedenen Ansichten eine erste Ausführungsform eines erfindungsgemäßen Konnektors 1 für medizinischen Wirkstoff enthaltende Behälter.

Der Konnektor 1 umfasst einen ersten Anschlussbereich 3 für den Anschluss eines ersten Behälters, ein Führungselement 4, ein Durchstechelement 5, eine Trennwand 6 und einen zweiten Anschlussbereich für den Anschluss eines zweiten Behälters. Der erste Anschlussbereich 3 ist hier für den Anschluss eines medizinischen Glasfläschchens ausgebildet. Der zweite Anschlussbereich 12 ist hier für den Anschluss eines flexiblen Beutels ausgebildet. Alternativ können der erste Anschlussbereich 3 und/oder der zweite Anschlussbereich 12 selbstverständlich auch für andere Behältnisse ausgebildet sein, beispielsweise Kunststoffflaschen oder Überleitsysteme.

Der Konnektor 1 umfasst ein Gehäuse 9 mit einem im Wesentlichen hohlzylinderförmig ausgebildeten, den ersten Anschlussbereich 3 ausbildenden Gehäuseabschnitt 10 und einem Gehäuseboden 11.Das Führungselement 4 ist als röhrchenförmiger Hohlkörper mit einem Kanal 7 für die Überführung eines medizinischen Wirkstoffes ausgebildet und im Zentrum des Gehäusebodens 11 mit dem Gehäuseboden 11 verbunden. Das eine Ende des Führungselementes endet am Gehäuseboden 11, das andere Ende ragt in den Anschlussbereich 3 und damit in den Innenbereich des Gehäuseabschnitts 3 hinein. Der in den Anschlussbereich 3 ragende Endabschnitt 8 des Führungselementes 4 ist angeschärft oder angespitzt, um eine Membran 14 eines im Anschlussbereich 3 anzuschließenden Behälters 2 zu durchstoßen. Gehäuseabschnitt 10 und Führungselement 4 sind axial einer gemeinsamen Symmetrieachse 15 angeordnet.

Des Weiteren umfasst der Konnektor 1 ein Verbindungsmittel 13, das den zweiten Anschlussbereich 12 ausbildet. Das Verbindungsmittel 13 ist schlauchförmig ausgebildet, längs der Symmetrieachse 15 angeordnet und mit einem Ende an der dem Anschlussbereich 3 gegenüberliegenden Rückseite des Gehäusebodens 11 verbunden. Am anderen Ende umfasst das Verbindungsmittel 13 zwei spiegelsymmetrisch ausgebildete Schweißlaschen 16 für das Verbinden des Verbindungsmittels 13 mit einem nicht näher dargestellten flexiblen Beutel aus Kunststoff. Des Weiteren umfasst das Verbindungsmittel 13 zwei seitlich, längs der Symmetrieachse 15 verlaufende Verstärkungsstege 17, die von dem Boden 11 bis zu den Schweißlaschen 16 spiegelsymmetrisch angeordnet verlaufen und den Zweck haben, das Verbindungsmittel zu versteifen.

Die Trennwand 6 ist in Form einer Membran ausgebildet. Sie ist zwischen dem ersten Anschlussbereich 3 und dem zweiten Anschlussbereich 12 angeordnet und verhindert im geschlossenen Zustand den Transport eines medizinischen Wirkstoffs zwischen diesen beiden Bereichen. Im speziellen ist die Trennwand 6 an dem dem ersten Anschussbereich 3 abgewandten Ende des Führungselementes 4 angeordnet und schließt bündig mit der Rückseite des Gehäusebodens 11 ab. Anstatt einer Anordnung der Trennwand 6 an einem Ende des Kanals 7 ist alternativ auch eine Anordnung der Trennwand 6 innerhalb des Kanals 7 des Führungselementes 4 möglich. Des Weiteren kann die Trennwand 6 derart angeordnet sein, dass diese sich vom Gehäuseboden 11 abhebt, beispielsweise deckelartig auf der Öffnung des Kanals 7 angeordnet ist. Des Weiteren kann die Trennwand 6 über mindestens eine Sollbruchstelle am Gehäuseboden 11 befestigt sein, so dass durch das Durchstechelement 5 die Trennwand 6 an der mindestens einen Sollbruchstelle aufbrechbar ist und auf diese Weise der Kanal 7 geöffnet wird. Ein Durchstechen der Trennwand 6 kann in diesem Falle entfallen.

Die Trennwand 6 ist als Wand mit ausreichend dünner Wandstärke ausgebildet, um von dem Durchstechelement 5 durchstochen und/oder aufgebrochen zu werden. In dieser Ausführungsform ist die Trennwand derart ausgebildet, dass diese nach dem Durchstechen/Aufbrechen bei einem Herausziehen des Durchstechelementes 5 geöffnet bleibt, was bevorzugt ist. Ebenfalls ist es möglich, die Trennwand 6 derart auszubilden, dass diese sich nach einem Herausziehen der Durchstechelementes 5 schliessen kann.

Gehäuse 9, Führungselement 4, Trennwand 6 und Verbindungsmittel 12 sind in diesem Ausführungsbeispiel einteilig und einstückig aus Kunststoff, hier aus PP oder einem Blend aus PP und SEBS, als Spritzgussteil hergestellt, wodurch der Konnektor besonders kostengünstig herstellbar ist. Grundsätzlich ebenfalls möglich ist ein mehrteiliger Aufbau und/oder ein Aufbau aus mehreren unterschiedlichen Materialien.

Das Durchstechelement 5 ist in diesem Ausführungsbeispiel als massives Stäbchen ausgebildet. Das Durchstechelement ist in dem Kanal 7 angeordnet und wird innerhalb des Kanals 7 vom Führungselement 4 axial beweglich geführt. Zwischen der Innenfläche des Kanals 7 und der Außenfläche des Durchstechelementes 5 besteht ein axial verlaufender Freiraum, der eine ausreichenden Querschnitt aufweist, um medizinischen Wirkstoff genügend schnell zwischen dem ersten Anschlussbereich 3 und dem zweiten Anschlussbereich 12 durchlassen zu können. Das Durchstechelement 5 hat hier ein kreuzförmiges Profil, das in seinen äußeren Abmaßen an dem Innendurchmesser des rohrförmigen Führungselementes 4 angepasst ist, so dass das Durchstechelement 5 nicht verkippt. Für das Durchstechelement 5 können auch andere Profile verwendet werden, beispielsweise Profile in Dreiecksform, Sternform, Quadratform und/oder Zylinderform. Das Durchstechelement 5 kann auch teilweise oder vollständig als Hohlkörper ausgebildet sein und der Transport des medizinischen Wirkstoffes teilweise oder vollständig durch das Durchstechelement 5 hindurch erfolgen. Auch das Führungselement 4 kann alternativ mit anderen Profilen ausgebildet sein, beispielsweise quadratisch oder rechteckförmig.

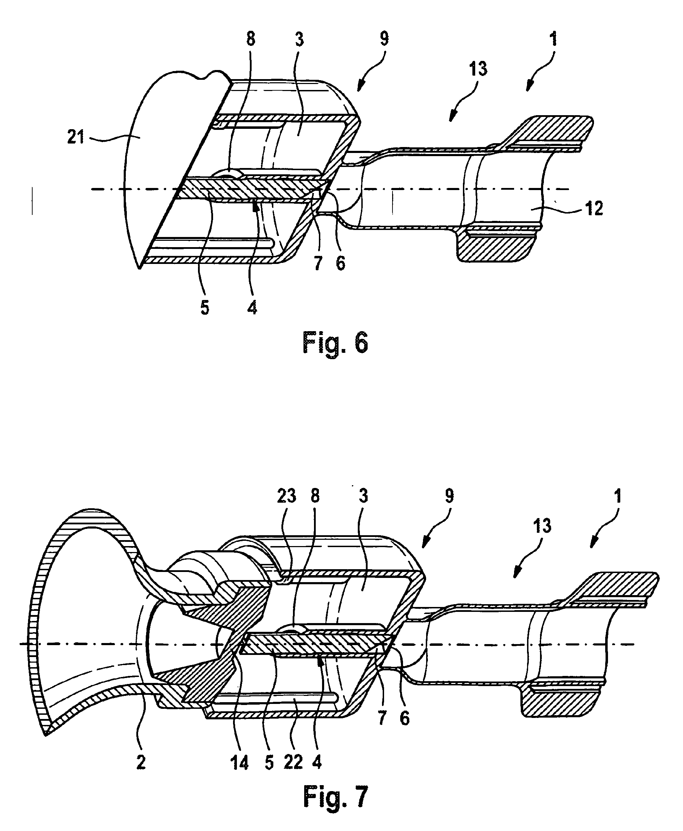

Des Weiteren ist das Durchstechelement 5 in einer Ausgangsstellung, in der das Durchstechelement 5 die Trennwand 6 nicht öffnet, lösbar vorfixiert. Hierzu umfasst das Durchstechelement 5 zwei gegenüberliegende Vorsprünge 18, die in der Ausgangsstellung in zwei korrespondierende Nuten 19 in dem Führungselement 4 lösbar eingreifen. Durch ausreichend Druck auf das Durchstechelement 5, wie er beispielsweise beim Anschließen einer Glasflasche in dem ersten Anschlussbereich 3 ausgeübt werden kann, kann das Durchstechelement 4 aus der Ausgangsstellung gelöst und in eine Endstellung überführt werden. Beim Überführen des Durchstechelementes 4 aus der Ausgangsstellung in die Endstellung öffnet das Durchstechelement 4 die Trennwand 6, siehe auch Fig. 6 bis 9. Das Führungselement 5 weist zwei weitere Nuten 20 auf, in die das Durchstechelement 4 nach Öffnen der Trennwand 6 einrasten kann und die verhindern, dass sich das Durchstechelement 4 ungewünscht vom Führungselement 4 löst. Alternativ kann am Durchstechelement 4 beispielsweise nur eine Nut oder weitere Nuten vorgesehen sein, wobei im Falle von mehreren Nuten diese beispielsweise versetzt angeordnet sein können. Als weitere Alternative kann das Durchstechelement beispielsweise durch ein Übermaß in dem Führungselement vorfixiert werden.

Zum leichteren Aufbrechen oder Durchstechen der Trennwand ist das Durchstechelement 5 an seinem der Trennwand 6 zugewandtem Ende angeschärft oder angespitzt. An seinem anderen, der Trennwand 6 abgewandten Ende bildet das Durchstechelement 5 eine Auflagefläche aus, um den Druck eines anzuschließenden Behälters möglichst großflächig aufzunehmen und um zu verhindern, dass das Durchstechelement 5 ungewünscht in den Behälter eindringt, beispielsweise einen Gummistopfen-Verschluss des Behälters durchsticht. Zu diesem Zweck ist in diesem Falle das der Trennwand 6 abgewandte Ende des Durchstechelements 5 als plane Fläche ausgebildet.

Das Durchstechelement 5 ist in diesem Ausführungsbeispiel einteilig und einstückig aus einem Kunststoff, vorzugsweise aus PP, PC oder PS, als Spritzgussteil hergestellt. Alternativ kann das Durchstechelement 5 auch mehrteilig und/oder aus anderen Materialien, beispielsweise auch aus einem Metall, und/oder aus mehreren Materialien bestehen.

Des Weiteren umfasst der Konnektor eine lösbare Abdeckfolie 21, die den ersten Anschlussbereich 3 verschließt und vor Verunreinigung, beispielsweise vor einer Kontamination durch unbeabsichtigte Berührung, schützt. Die Abdeckfolie kann beispielsweise eine manuell abziehbare Aluminiumfolie oder Kunststofffolie sein.

Die Figuren 6 bis 9 zeigen den Anschluss eines Glasfläschchens 2 an den Konnektor 1.

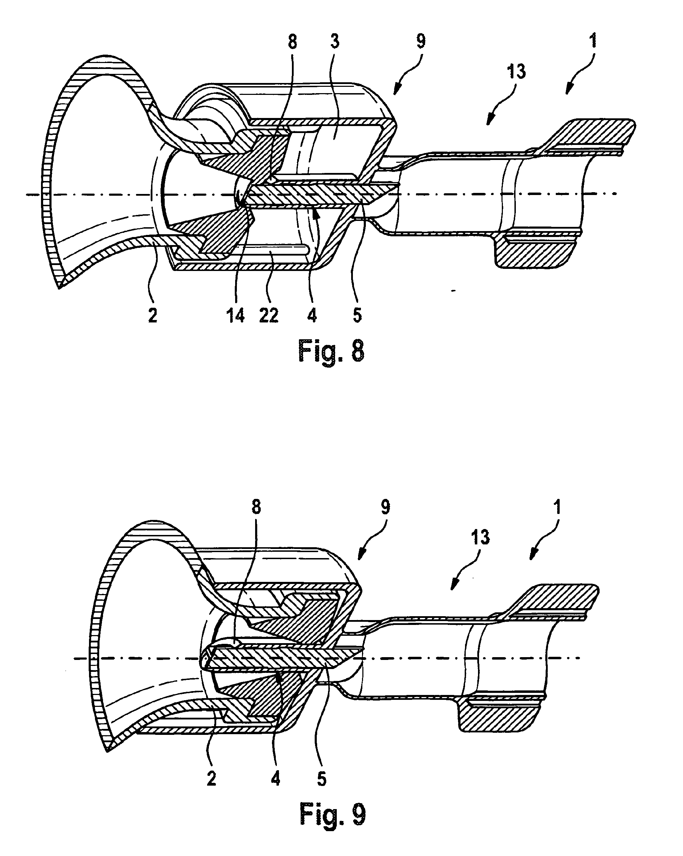

Fig. 6 zeigt den Konnektor 1 im Ausgangszustand. Zum Anschließen einer Glasflasche 2 wird zunächst die Abdeckfolie 21 manuell abgezogen. Anschließend wird die Glasflasche mit ihren Hals in den ersten Anschlussbereich 3 eingeführt, siehe Fig. 7. Beim Einführen der Glasflasche 2 übt die Glasflasche 2 einen auf das über das Führungselement 4 hinausragende, der Glasflasche 2 zugewandtem Ende des Durchstechelements 5 axialen Druck auf das Durchstechelement 5 auf, der das Durchstechelement 5 mit seinem der Trennwand 6 zugewandten Ende gegen die Trennwand 6 drückt, bis das Durchstechelement 5 durch den Druck die Trennwand 6 durchbrochen hat. Durch Weiteres Einschieben des Behälters 2 wird das Durchstechelement 5 axial verschoben, bis der Behälter 2 eine vorgelagerte Behälterandockposition erreicht, siehe Figur 8. Das Gehäuse 9 umfasst mehrere axial verlaufende, an der Innenseite des Gehäuseabschnitts 10 verlaufende Klemmstege 22, die die Glasflasche beim Einführen in den Anschlussbereich durch Klemmen mit dem Konnektor 1 verbinden. In der Behälterandockposition ist die im Hals der Behälter 2 angeordnete Membran 14, hier ein Gummistopfen, noch nicht durchstoßen, so dass in dieser Stellung eine Überführung eines medizinischen Wirkstoffes noch unterbunden ist. Insbesondere kann der Behälter 2 vom Konnektor 1 gelöst werden, ohne dass dieser bereits geöffnet wurde. Letzteres ist insbesondere dann von Vorteil, wenn der Behälter 2 teure Wirkstoffe enthält.

Beim Weiteren Einschieben des Behälters 2 in den Anschlussbereich 3 wird die Membran 14 durch den kanülenartigen Endabschnitt 8 des Führungselementes 4 durchstoßen, siehe Figur 9. Ein sich in dem Behälter 2 befindender Wirkstoff, beispielsweise eine Flüssigkeit oder ein Pulver, kann nun mittels dem Konnektor 1 überführt und beispielsweise verdünnt und/oder aufgelöst werden, beispielsweise in einem mit dem Verbindungsmittel 13 verbundenen flexiblen Beutel, oder umgekehrt ein Wirkstoff in den Behälter 2 geleitet werden. Beim Weiteren Einschieben des Behälters 2 erreicht der Behälter 2 eine Endposition, in der ein oder mehrere Vorsprünge 23 an der Innenseite des Gehäuseabschnittes 10, siehe beispielsweise Fig. 8, den Behälter 2 an seinem Hals formschlüssig hintergreifen und ein Herausrutschen des Behälters 2 aus dem ersten Anschlussbereich 3 des Konnektors 1 verhindern.

Im Anschlussbereich 3 kann ein weiterer Hinterschnitt vorgesehen sein, beispielsweise durch zusätzliche Vorsprünge, der den Behälter 2 bereits in der Andockposition unter Ausbildung eines Formschlusses fixiert. Die Klemmstege 22 können in dieser Variante ebenfalls vorhanden sein, alternativ kann auf diese aber auch verzichtet werden. Ein solcher zweiter Hinterschnitt erleichtert das Verbinden von Behältern mit unterschiedlichen Abmaßen mit dem Konnektor 1.

Der Behälter 2 kann in der Andockposition am Konnektor vormontiert sein. Der vormontierte Behälter 2, der Konnektor 1 und ein mit dem Konnektor 1 verbundenes Behältnis, insbesondere ein Beutel, können in einem Umbeutel als Set eingeschlossen sein. Dies ermöglicht ein sicheres Mischen der Komponenten außerhalb des Laminar-Flow-Bereiches ohne das Risiko einer Kontamination.

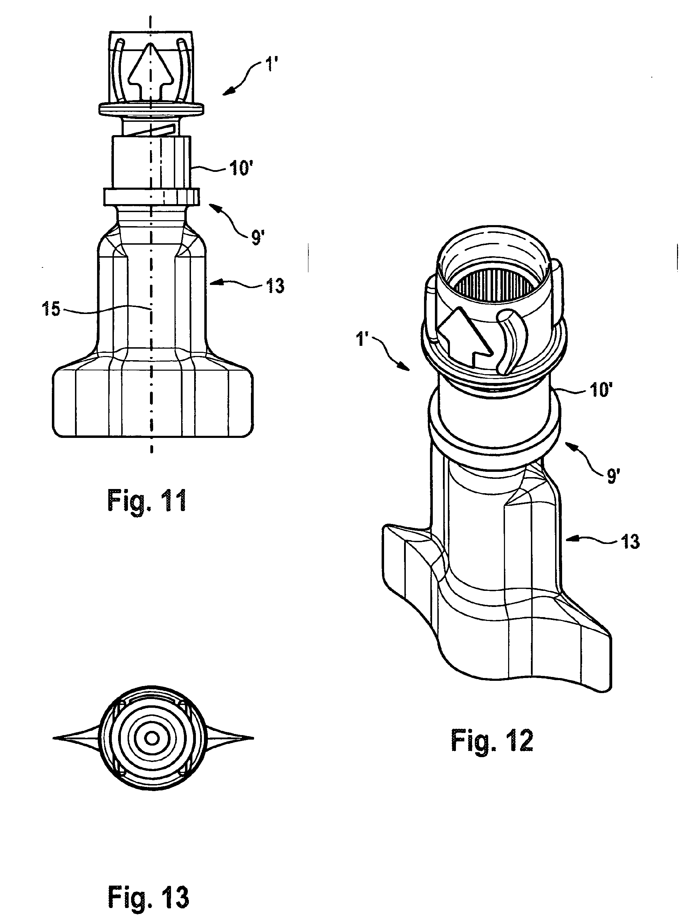

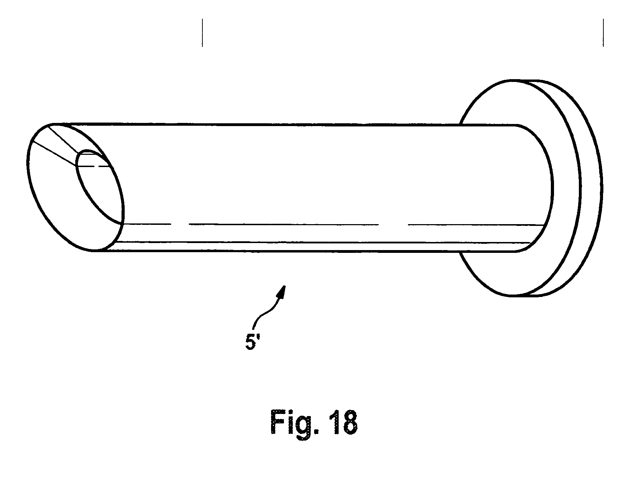

Die Figuren 11 bis 18 zeigen eine zweite Ausführungsform eines erfindungsgemäßen Konnektors 1' für medizinischen Wirkstoff enthaltende Behälter in verschiedenen Ansichten. Elemente oder Bauteile, die denen der ersten Ausführungsform entsprechen, wurden mit denselben Bezugszeichen gekennzeichnet, die für die Kennzeichnung der Bauteile der ersten Ausführungsform verwendet wurden.

Der Konnektor 1' gemäß der zweiten Ausführungsform umfasst einen ersten Anschlussbereich 3', ein Führungselement 4', ein Durchstechelement 5', eine Trennwand 6 und einen zweiten Anschlussbereich 12. Der zweite Anschlussbereich 12 wird durch ein Verbindungsmittel 13 ausgebildet, das dem der ersten Ausführungsform entspricht. Der erste Anschlussbereich 3' ist, abweichend von der ersten Ausführungsform, als Luer-Anschluss eines Luer-Locks (ISO 594/1) ausgebildet, hier als Luer-Male-Teil. Anschlussbereich 3', Führungselement 4', Durchstechelement 5' und Verbindungsmittel 13 sind entlang der gemeinsamen Symmetrieachse 15 angeordnet.

Der Konnektor 1' umfasst ein Gehäuse 9' mit einem im Wesentlichen zylinderförmigen Gehäuseabschnitt 10' mit einem Innengewinde und einem Gehäuseboden 11', die den Anschlussbereich 3' ausbilden. Das Führungselement 4' ist als hohler Außenkegel ausgebildet und ragt in den Anschlussbereich 3' hinein. Durch die Ausbildung als Hohlkörper umfasst das Führungselement 4' einen innen liegenden Kanal 7, der für die Überführung eines medizinischen Wirkstoffs vorgesehen ist. Das Führungselement 4' ist im Zentrum des Gehäusebodens 11' mit dem Gehäuseboden 11' verbunden.

Die Trennwand 6 ist entsprechend der Trennwand 6 gemäß der ersten Ausführungsform in der Ebene des Gehäusebodens 11' angeordnet und entsprechend ausgebildet. Im geschlossenen Zustand unterbindet die Trennwand 6 einen Transport eines medizinischen Wirkstoffs zwischen dem ersten Anschlussbereich 3' und dem zweiten Anschlussbereich 12.

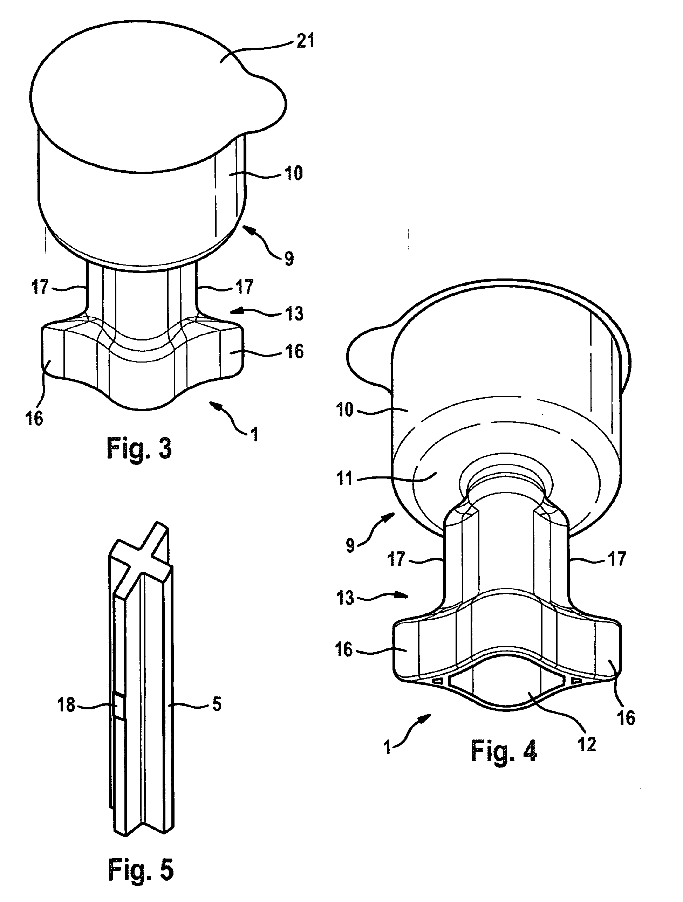

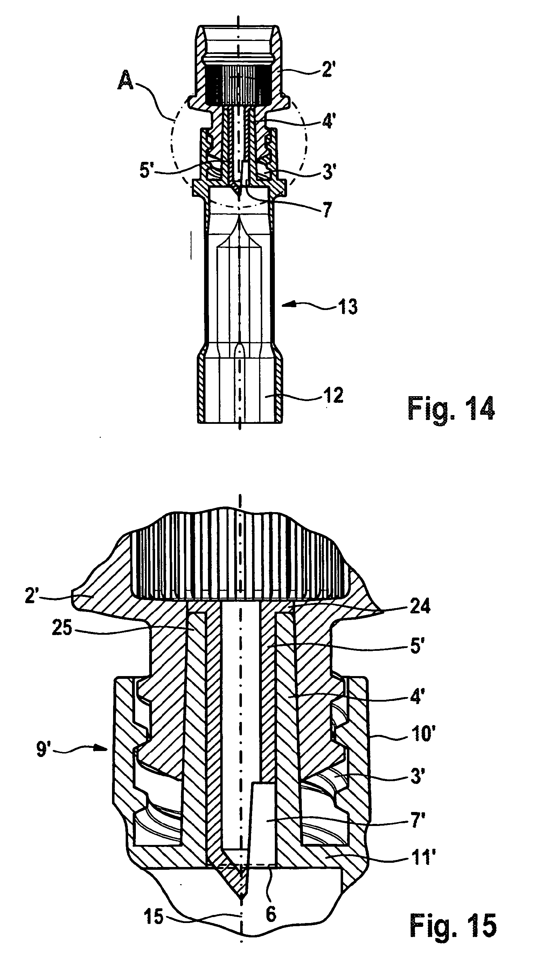

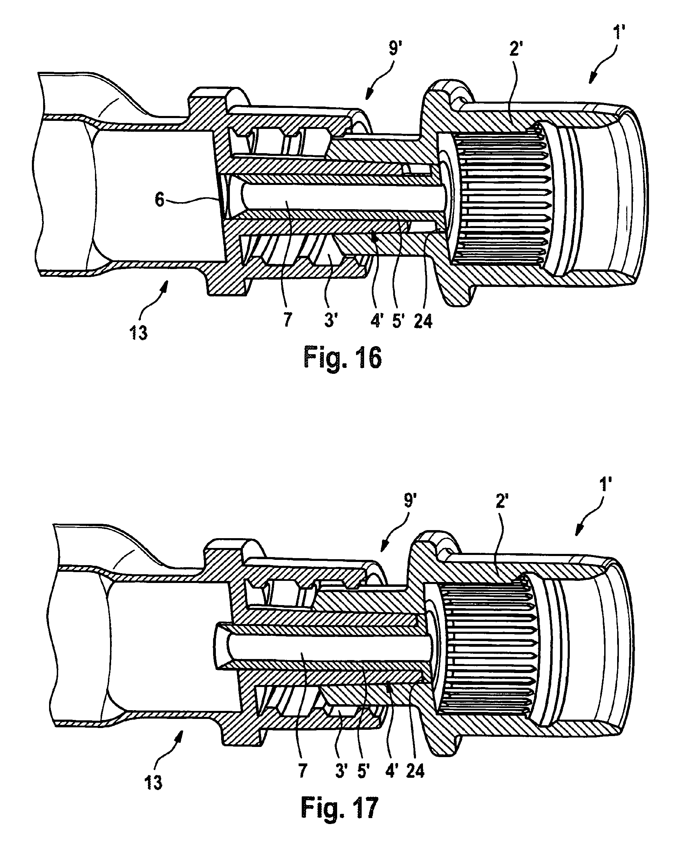

Das Durchstechelement 5' ist röhrchenförmig als Hohlkörper ausgeführt. Das Durchstechelement 5' ist abschnittsweise in dem Kanal 7 angeordnet und wird innerhalb des Kanals 7 vom Führungselement 4' axial beweglich geführt. Der Außendurchmesser des Durchstechelementes 5' ist in dem geführten Abschnitt an den Innendurchmesser des Führungselementes 4' zur möglichst spielfreien Lagerung angepasst. An seinem dem ersten Anschlussbereich 3' zugewandtem Ende weist das Durchstechelement 5' einen Kragen 24 auf, siehe Fig. 5, an seinem dem ersten Anschlussbereich 3' abgewandtem Ende ist das Durchstechelement 5' zum leichteren Durchtrennen der Trennwand 6 angeschärft oder angespitzt. In einer Ausgangsstellung, in der das Durchstechelement 5' die Trennwand 6 nicht öffnet, siehe Fig. 16, ist der Kragen 24 von dem Führungselement 4' beabstandet, in einer Endstellung, in der das Durchstechelement 5' die Trennwand 6 zum Überführen eines medizinischen Wirkstoffes öffnet, liegt der Kragen 24 auf dem oberen Rand 25 des Führungselementes 4' auf, siehe Fig. 15 und 17, wodurch ein weiteres Einschieben des Durchstechelementes 5' in das Führungselement 4' blockiert ist. Alternativ kann das Durchstechelement 5' auch als massiver Körper ausgebildet sein, beispielsweise mit einem kreuzförmigen, sternförmigen, dreiecksförmigen oder ähnlichen Profil. In diesem Fall ist ein zwischen der Innenfläche des Führungselementes 4' und der Außenfläche des Durchstechetementes 5' verbleibender Freiraum für den Transport des medizinischen Wirkstoffs nutzbar.

Gehäuse 9', Führungselement 4', Trennwand 6 und Verbindungsmittel 6 sind in diesem Ausführungsbeispiel als einteiliges und einstückiges Spritzgussteil aus einem Kunststoff, vorzugsweise PP oder ein PP-SEBS-Blend, hergestellt. Prinzipiell wäre auch ein mehrteiliger Aufbau und die Verwendung anderer und/oder unterschiedlicher Materialien möglich. Das Durchstechelement 5' ist ebenfalls ein einteiliges und eintstückiges Spritzgussteil aus Kunststoff, vorzugsweise PP. PC oder PS . Auch hier wäre ein mehrteiliger Aufbau und die Verwendung anderer und/oder unterschiedlicher Materialien als Alternative grundsätzlich möglich.

Die dargestellten Figuren zeigen neben dem Konnektor 1' ein Element eines Behälters, hier ein Luer-Female-Teil 2', das an dem Konnektor 1' angeschlossen wird bzw. angeschlossen ist. Das Luer-Female-Teil 2' kann beispielsweise Bestandteil einer Spritze oder eines Überleitsystems sein.

Fig. 16 zeigt den Zustand nach dem Ansetzen des Luer-Female-Teils 2' und vor dem Verriegeln des Luer-Female-Teils 2' mit dem Luer-Male-Teil des Konnektors 1'. In dieser Stellung ist das Führungselement 4' mit dem hervorstehenden Durchstechelement 5' bereits teilweise in den Innenkonus des Luer-Female-Teils 2' eingeführt. Der Außendurchmesser des Kragens 24 des Durchstechelementes 5' ist an den Durchmesser des Innenkonus des Luer-Female-Teils 2' angepasst, so dass der Kragen 24 nicht durch den Innenkonus vollständig hindurchsteckbar ist, sondern zuvor durch den sich verjüngenden Innenkonus an seinem Außenrand eingeklemmt wird, hier nahezu am Ende des Innenkonus. Bei einer Verriegelung des Luer-Female-Teils 2' mit dem Male-Teil, das durch das Gehäuse 9' ausgebildet wird, verschiebt sich das Luer-Female-Teil 2' axial in Richtung des Konnektors 1'. Das am Kragen 24 geklemmte Durchstechelement 5' wird bei dieser Bewegung vom Female-Teil 2' mitgeführt, und die Trennwand 6 durch das angeschärfte oder angespitzte Ende des Durchstechelementes 5' aufgebrochen bzw. durchstoßen und geöffnet, siehe Fig. 17. Die Verbindung zwischen dem ersten Anschlussbereich 3' und dem zweiten Anschlussbereich 12 ist nun geöffnet, was den Transport eines medizinischen Wirkstoffes zwischen einem im Anschlussbereich 12 mit dem Konnektor 1' angeschlossenen flexiblen Beutel oder einem anderem Behältnis mit einem durch das Luer-Female-Teil 2' angeschlossenen Behältnis ermöglicht.

Der Anschlussbereich 3' des Konnektors 1' mit Durchstechelement 5' kann zum Schutz gegen Verunreinigungen und/oder zum Schutz gegen ein ungewolltes Öffnen der Trennwand 6 durch eine Bewegung des Durchstechelementes 5' durch eine manuell ablösbare Kappe (nicht dargestellt) bedeckt sein. Des Weiteren ist das Durchstechelement 5' in seiner ausgezogenen Ausgangsstellung vorzugsweise vorfixiert, beispielsweise mittels einer Feder-Nut-Verbindung ähnlich der der ersten Ausführungsform. Auch eine Fixierung des Durchstechelementes 5' in der Endstellung ist möglich, beispielsweise um ein Herausfallen des Durchstechelementes 5' bei Lösen des Female-Parts 2' zu verhindern.

Die erfindungsgemäßen Konnektoren 1, 1' besitzen den Vorteil, dass diese kostengünstig herstellbar sind. Des Weiteren kann in einfacher Weise sicher und schnell eine sterile Verbindung zwischen zwei Behältern manuell hergestellt werden. Zur schnellen Überführung eines medizinischen Wirkstoffs können Führungselement 4, 4' und Durchstechelement 5, 5' mit geeigneten Profilen ausgebildet werden.

## Patentansprüche

1. Konnektor (1, 1') für medizinischen Wirkstoff enthaltende Behälter, umfassend einen ersten Anschlussbereich (3, 3') für den Anschluss eines ersten Behälters (2, 2'), ein Führungselement (4, 4'), ein Durchstechelement (5, 5') und eine Trennwand (6), wobei das Führungselement (4, 4') einen für die Überführung eines medizinischen Wirkstoffes ausgebildeten Kanal (7) aufweist, das Durchstechelement (5, 5') zumindest abschnittsweise in dem Kanal (7) angeordnet ist und innerhalb des Kanals (7) vom Führungselement (4, 4') axial beweglich geführt wird, und durch das Anschließen eines Behälters (2, 2') im ersten Anschlussbereich (3, 3') das Durchstechelement (5, 5') von einer Ausgangsstellung, in der das Durchstechelement (5, 5') die Trennwand (6) nicht öffnet, in eine Endstellung, in der das Durchstechelement (5, 5') die Trennwand (6) zum Überführen eines medizinischen Wirkstoffs öffnet, bewegbar ist, **dadurch gekennzeichnet, dass** das Führungselement (4, 4') einen Endabschnitt (8) aufweist, der zum Durchstechen und/oder Aufbrechen einer Membran (14) eines in den ersten Anschlussbereich (3, 3') anschließbaren Behälters (2, 2') ausgebildet ist und/oder dass der Konnektor (1, 1') ein Gehäuse (9, 9') umfasst mit einem den ersten Anschlussbereich (3, 3') ausbildenden Gehäuseabschnitt (10, 10') und einem Gehäuseboden (11, 11'), wobei das Führungselement (4, 4') am Gehäuseboden (11, 11') mit dem Gehäuse (9, 9') verbunden ist.

2. Konnektor (1, 1') nach Anspruch 1, wobei das Durchstechelement (5, 5') mit seinem dem ersten Anschlussbereich (3, 3') zugewandtem Ende über das Führungselement (4, 4') hinausragt.

3. Konnektor (1, 1') nach einem der vorhergehenden Ansprüche, wobei das Durchstechelement (5, 5') und das Führungselement (4, 4') derart ausgebildet sind, dass zwischen einer Außenfläche des Durchstechelementes (5, 5') und einer den Kanal (7) bildenden Innenfläche des Führungselementes (4, 4') ein Freiraum für die Überführung eines medizinischen Wirkstoffes verbleibt.

4. Konnektor (1, 1') nach einem der vorhergehenden Ansprüche, wobei das Durchstechelement (5, 5') als massiver Körper und/oder als Hohlkörper ausgebildet ist.

5. Konnektor (1, 1') nach einem der vorhergehenden Ansprüche, wobei das Durchstechelement (5, 5') zumindest teilweise, vorzugsweise vollständig, aus einem Kunststoff besteht, bevorzugt aus Polypropylen, Polycarbonat oder Polystyrol.

6. Konnektor (1, 1') nach einem der vorhergehenden Ansprüche, wobei die Trennwand (6) innerhalb des Kanals (7) oder an einem Ende des Kanals (7) angeordnet ist.

7. Konnektor (1, 1') nach einem der vorhergehenden Ansprüche, wobei die Trennwand (6) an dem dem ersten Anschlussbereich (3, 3') abgewandten Ende des Durchstechelementes (5, 5') angeordnet ist.

8. Konnektor (1, 1') nach einem der vorhergehenden Ansprüche, wobei das Durchstechelement (5, 5') in der Ausgangsstellung lösbar vorfixiert ist.

9. Konnektor (1, 1') nach Anspruch 1, wobei der Konnektor (1, 1') derart ausgebildet ist, dass beim Anschließen an den ersten Anschlussbereich (3, 3') und Überführen des Behälters (2, 2') in eine vorgelagerte Behälterandockposition zunächst die Trennwand (6) durch das Durchstechelement (5, 5') geöffnet wird, und beim Überführen des Behälters (2, 2') in eine Behälterendposition die Membran (14) des Behälters (2, 2') durch das Führungselement (4, 4') geöffnet wird.

10. Konnektor (1, 1') nach einem der vorhergehenden Ansprüche, zusätzlich umfassend einen zweiten Anschlussbereich (12) für den Anschluss eines zweiten Behälters, wobei die Trennwand (6) derart angeordnet ist, dass diese im geschlossenen Zustand den Transport eines medizinischen Wirkstoffs zwischen dem ersten Anschlussbereich (3, 3') und dem zweiten Anschlussbereich (12) unterbindet.

11. Konnektor (1, 1') nach Anspruch 10 in Verbindung mit Anspruch 9, wobei der zweite Anschlussbereich (3, 3') durch ein mit dem Gehäuseboden (11, 11') verbundenes Verbindungsmittel (13) ausgebildet wird.

12. Konnektor (1, 1') nach Anspruch 11, wobei das Gehäuse (9, 9') und das Verbindungsmittel (13) einteilig, vorzugsweise einstückig sind.

13. Konnektor (1, 1') nach einem der vorhergehenden Ansprüche, wobei der erste Anschlussbereich (3, 3') für den Anschluss einer Glasflasche oder als Luer-Anschluss ausgebildet ist.

## Claims

1. Connector (1, 1') for containers containing medicinal active ingredient, comprising a first connection region (3, 3') for the connection of a first container (2, 2'), a guide element (4, 4'), a piercing element (5, 5') and a partition wall (6), wherein the guide element (4, 4') has a duct (7) designed for transferring a medicinal active ingredient, the piercing element (5, 5') is arranged, at least in certain sections, in the duct (7) and is axially movably guided within the duct (7) by the guide element (4, 4'), and, by connecting a container (2, 2') in the first connection region (3, 3'), the piercing element (5, 5') can be moved from a starting position, in which the piercing element (5, 5') does not open the partition wall (6), into an end position, in which the piercing element (5, 5') opens the partition wall (6) in order to transfer a medicinal active ingredient, **characterized in that** the guide element (4, 4') has an end section (8) which is designed to pierce and/or break open a membrane (14) of a container (2, 2') which can be connected into the first connection region (3, 3'), and/or, **in that** the connector (1, 1') comprises a housing (9, 9') with a housing section (10, 10'), forming the first connection region (3, 3'), and a housing base (11, 11'), wherein the guide element (4, 4') is connected to the housing (9, 9') at the housing base (11, 11').

2. Connector (1, 1') according to Claim 1, wherein the piercing element (5, 5') projects by its end facing the first connection region (3, 3') beyond the guide element (4, 4').

3. Connector (1, 1') according to one of the preceding claims, wherein the piercing element (5, 5') and the guide element (4, 4') are designed in such a way that a clearance for transferring a medicinal active ingredient remains between an outer face of the piercing element (5, 5') and an inner face, forming the duct (7), of the guide element (4, 4').

4. Connector (1, 1') according to one of the preceding claims, wherein the piercing element (5, 5') is designed as a solid body and/or as a hollow body.

5. Connector (1, 1') according to one of the preceding claims, wherein the piercing element (5, 5') consists at least partially, preferably completely, of a plastic, preferably of polypropylene, polycarbonate or polystyrene.

6. Connector (1, 1') according to one of the preceding claims, wherein the partition wall (6) is arranged within the duct (7) or at an end of the duct (7).

7. Connector (1, 1') according to one of the preceding claims, wherein the partition wall (6) is arranged at the end of the piercing element (5, 5') facing away from the first connection region (3, 3').

8. Connector (1, 1') according to one of the preceding claims, wherein the piercing element (5, 5') is releasably prefixed in the starting position.

9. Connector (1, 1') according to Claim 1, wherein the connector (1, 1') is designed such that, when connecting to the first connection region (3, 3') and transferring the container (2, 2') into an upstream container docking position, at first the partition wall (6) is opened by the piercing element (5, 5'), and, when transferring the container (2, 2') into a container end position, the membrane (14) of the container (2, 2') is opened by the guide element (4, 4').

10. Connector (1, 1') according to one of the preceding claims, additionally comprising a second connection region (12) for the connection of a second container, wherein the partition wall (6) is arranged such that in the closed state it prevents the transport of a medicinal active ingredient between the first connection region (3, 3') and the second connection region (12).

11. Connector (1, 1') according to Claim 10 in conjunction with Claim 9, wherein the second connection region (3, 3') is formed by a connecting means (13) connected to the housing base (11, 11').

12. Connector (1, 1') according to Claim 11, wherein the housing (9, 9') and the connecting means (13) are one-part, preferably integral.

13. Connector (1, 1') according to one of the preceding claims, wherein the first connection region (3, 3') is designed for the connection of a glass bottle or as a Luer connection.

## Revendications

1. Connecteur (1, 1') pour récipient contenant un principe actif médicinal, comportant une première région de raccordement (3, 3') pour le raccordement d'un premier récipient (2, 2'), un élément de guidage (4, 4'), un élément de perforation (5, 5') et une paroi de séparation (6), l'élément de guidage (4, 4') comprenant un canal (7) réalisé pour le transfert d'un principe actif médicinal, l'élément de perforation (5, 5') étant disposé au moins en partie dans le canal (7) et étant guidé de manière axialement mobile par l'élément de guidage (4, 4') à l'intérieur du canal (7) et, par le raccordement d'un récipient (2, 2') dans la première région de raccordement (3, 3'), l'élément de perforation (5, 5') pouvant être déplacé à partir d'une position initiale, dans laquelle l'élément de perforation (5, 5') n'ouvre pas la paroi de séparation (6), jusqu'à une position finale, dans laquelle l'élément de perforation (5, 5') ouvre la paroi de séparation (6) pour transférer un principe actif médicinal, **caractérisé en ce que** l'élément de guidage (4, 4') comprend une portion d'extrémité (8) qui est réalisée pour perforer et/ou éventrer une membrane (14) d'un récipient (2, 2') pouvant être raccordé dans la première région de raccordement (3, 3'), et/ou **en ce que** le connecteur (1, 1') comporte un boîtier (9, 9') comprenant une portion de boîtier (10, 10') formant la première région de raccordement (3, 3') et un fond de boîtier (11, 11'), l'élément de guidage (4, 4') étant relié au boîtier (9, 9') au niveau du fond de boîtier (11, 11').

2. Connecteur (1, 1') selon la revendication 1, dans lequel l'élément de perforation (5, 5') fait saillie, par son extrémité tournée vers la première région de raccordement (3, 3'), au-delà de l'élément de guidage (4, 4').

3. Connecteur (1, 1') selon l'une quelconque des revendications précédentes, dans lequel l'élément de perforation (5, 5') et l'élément de guidage (4, 4') sont réalisés de telle sorte qu'un espace libre pour le transfert d'un principe actif médicinal demeure entre une surface extérieure de l'élément de perforation (5, 5') et une surface intérieure de l'élément de guidage (4, 4') formant le canal (7).

4. Connecteur (1, 1') selon l'une quelconque des revendications précédentes, dans lequel l'élément de perforation (5, 5') est réalisé sous forme de corps plein et/ou sous forme de corps creux.

5. Connecteur (1, 1') selon l'une quelconque des revendications précédentes, dans lequel l'élément de perforation (5, 5') est constitué au moins partiellement, de préférence entièrement, d'un plastique, de préférence de polypropylène, de polycarbonate ou de polystyrène.

6. Connecteur (1, 1') selon l'une quelconque des revendications précédentes, dans lequel la paroi de séparation (6) est disposée à l'intérieur du canal (7) ou à une extrémité du canal (7).

7. Connecteur (1, 1') selon l'une quelconque des revendications précédentes, dans lequel la paroi de séparation (6) est disposée à l'extrémité de l'élément de perforation (5, 5') opposée à la première région de raccordement (3, 3').

8. Connecteur (1, 1') selon l'une quelconque des revendications précédentes, dans lequel l'élément de perforation (5, 5') est préfixé de manière amovible dans la position initiale.

9. Connecteur (1, 1') selon la revendication 1, dans lequel le connecteur (1, 1') est réalisé de telle sorte que, lors du raccordement à la première région de raccordement (3, 3') et du transfert du récipient (2, 2') à une position d'arrimage de récipient située en amont, la paroi de séparation (6) est tout d'abord ouverte par l'élément de perforation (5, 5') et, lors du transfert du récipient (2, 2') à une position finale de récipient, la membrane (14) du récipient (2, 2') est ouverte par l'élément de guidage (4, 4').

10. Connecteur (1, 1') selon l'une quelconque des revendications précédentes, comportant en outre une deuxième région de raccordement (12) pour le raccordement d'un deuxième récipient, la paroi de séparation (6) étant disposée de telle sorte que celle-ci empêche, dans l'état fermé, le transport d'un principe actif médicinal entre la première région de raccordement (3, 3') et la deuxième région de raccordement (12).

11. Connecteur (1, 1') selon la revendication 10 en association avec la revendication 9, dans lequel la deuxième région de raccordement (3, 3') est réalisée par un moyen de liaison (13) relié au fond de boîtier (11, 11').

12. Connecteur (1, 1') selon la revendication 11, dans lequel le boîtier (9, 9') et le moyen de liaison (13) sont réalisés d'un seul tenant, de préférence d'une seule pièce.

13. Connecteur (1, 1') selon l'une quelconque des revendications précédentes, dans lequel la première région de raccordement (3, 3') est réalisée pour le raccordement d'une bouteille en verre ou sous forme de raccord Luer.
